# EUROPEAN PATENT SPECIFICATION

(11) **EP 0 499 690 B1**
(45) Date of publication and mention of the grant of the patent: **09.11.1994**
(21) Application number: 91116495.2
(22) Date of filing: 27.09.1991
(51) Int. Cl.: B05B 1/34, B05B 11/00

(54) **Nasal dispenser for atomizing pharmaceutical substances**
Nasaler Spender zur Zerstäubung von Arzneimitteln
Dispensateur nasal pour atomiser des produits pharmaceutiques

(30) Priority: 21.02.1991 IT MI043691
(43) Date of publication of application: 26.08.1992
(73) Proprietor: ELETTRO PLASTICA S.p.A., I-20089 Quinto de Stampi-Rozzano (Milano) (IT)
(72) Inventor: Marelli, Luciano, I-20089 Rozzano, MI (IT); Marelli, Andrea, I-20089 Rozzano, MI (IT)
(74) Representative: Giambrocono, Alfonso, Dr. Ing.

(56) References cited:
- EP-A- 0 131 501
- EP-A- 0 412 524
- US-A- 3 471 092

## Description

This invention relates to a nasal dispenser for atomizing pharmaceutical substances.

The use of nasally administered pharmaceutical substances is becoming increasingly more widespread. For this purpose, such substances are contained in a container or bottle, on the mouth of which there is mounted a pump or a dispensing valve with a hollow stem which when operated causes the substance to emerge through the stem cavity.

Nasal dispensers must be thin and long to enable them to be inserted through the nostrils and into the nasal cavities into which the pharmaceutical substance is to be delivered in atomized form. Such dispensers are therefore transversed by a channel of some considerable length. As the pharmaceutical substances are often very costly (for example calcitonin), the free volume of this channel must be as small as possible, so that the smallest possible quantity of substance remains trapped within it, to possibly be lost especially during the initial stage of pump operation (priming), when it is desirable for the liquid emerging from the pump stem to already fill the entire free volume of the dispenser channel on initial operation of the pump.

As dispensers are produced in large number by automatic machines, it is practically impossible to form long channels of very small cross-section.

In the patent EP-B-0 131 501 it is proposed to form the dispenser channel of fairly large uniform cross-section, and then to insert into it a constant-section peg as long as the channel and having a flattened longitudinal portion on its outer surface, such as to define a small-dimension passage together with the opposing surface of the channel. This nasal dispenser has however drawbacks deriving from the fact that only a very small expansion chamber for the pressurized substance passing through the channel is provided, between the free end of the peg and the opposing wall of the dispenser where the discharge hole for the atomized substance is located, and the fact that the free end of the dispenser where this discharge hole for the atomized substance is provided has to be shaped to define a hole at and about which there is a circular recess from which tangential channels extend to impress a vortex rotary motion on the particles, which then emerge from the dispenser through said hole which, being completely free, partly reduces the desired vortex motion of the particles. As said recess and the relative tangential channels have very small dimensions and are provided within the dispenser at the bottom of the long (and fairy small) bore which traverses it, it is difficult to form them using automatic machines of high production rate without a high level of rejects.

The lack of an additional expansion chamber within the dispenser channel is a drawback because the liquid substance delivered via the stem of the pump on which the dispenser is mounted cannot be atomized to the total and complete extent which would be desirable, with consequent spraying of relatively large droplets of the substance.

This problem is well known in the state of the art, as is apparent for example from US patents US-A-2 775 483 and US-A-3 471 092 which describe dispensers traversed by channels of relatively large section and housing profiled elements which define intermediate expansion chambers along the length of the dispensers between successive regions of limited cross-section for the passage of the liquid being dispensed, such profiled elements having a rather complicate structure and being provided with necessarily very small longitudinal channels extending along their periphery.

An object of the present invention is therefore to provide a nasal dispenser which can be produced with considerable ease and rapidity by automatic machines, and which effectively provides perfect atomization of the substance emerging from the dispenser.

This and further objects are attained by a nasal dispenser for atomizing pharmaceutical substances which is traversed by a longitudinal channel having an open end into which the stem of a pump or the like can be inserted, and provided at its other end with a wall provided with a hole, said channel housing an elongate profiled element the cross-sections of which are smaller than their surrounding cross-sections of the channel into which said element is inserted, wherein said channel has a cross-section which decreases stepwise towards said wall, the cross-sections of the elongate element being smaller than their surrounding cross-sections of the channel into which it is inserted, said element defining within the channel at least one intermediate expansion chamber, from the free end of the elongate element in proximity to said wall there projecting a peg which extends through the centre of the hole in said wall to leave a free space for the emergence of the atomized substance, in said end of the elongate element there being provided channels substantially tangential to the peg and opening into the periphery of the elongate element, characterised in that said profiled element has circular cross-sections decreasing stepwise from one end to the other along its length, and in that the cross-sections of said channel are in the form of lobes within its larger cross-section portion into which said element is forced, the length of said portion of said element inserted into the smaller cross-section portion of said channel being greater than the length of such channel portion.

The structure and characteristics of the nasal dispenser according to the present invention will be more apparent from the description of a preferred but non-limiting embodiment thereof given hereinafter with reference to the accompanying drawing, in which:
Figure 1 is a longitudinal section through the dispenser;
Figure 2 is a perspective view to a very enlarged scale of the profiled element inserted into the dispenser; and
Figures 3 to 6, also to an enlarged scale, represent cross-sections through the dispenser taken on the lines 3-3, 4-4, 5-5 and 6-6 respectively of Figure 1.

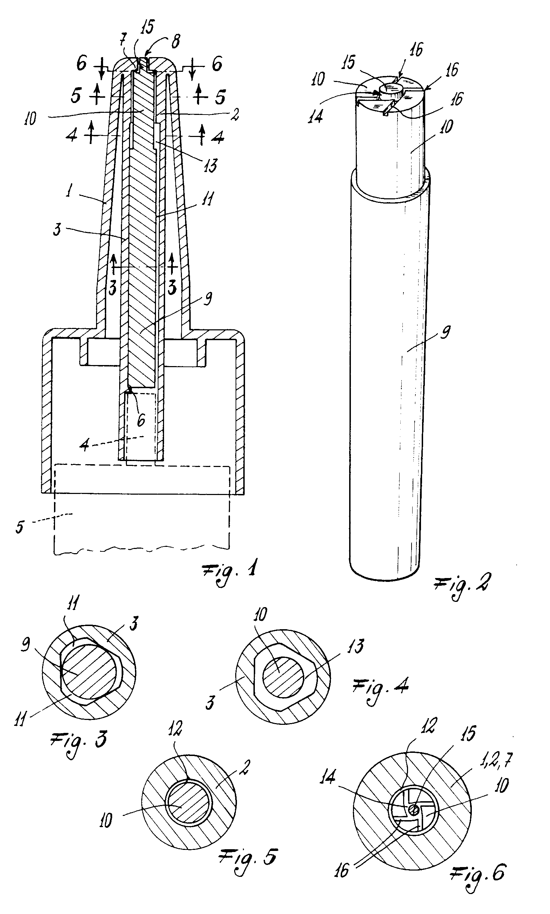

The nasal dispenser shown in Figure 1 comprises a slightly conical elongate outer wall 1 and an inner tubular wall consisting of two consecutive wall portions 2 and 3, defining a single elongate channel (of which the portion delimited by the wall portion 2 has a cross-section less than the channel portion delimited by the wall portion 3), the channel portion delimited by the wall portion 2 having a circular cross-section, whereas the channel portion delimited by the wall portion 3 is shaped in the form of lobes.

The free end of the wall portion 3 can be mounted on a stem 4 forming part of a manually operated pump or the like fitted to a container 5 containing the liquid substance to be dispensed by the dispenser in order to be then inhaled by the user.

It can be seen from Figure 1 that inwards from the tubular wall 3 there projects a tooth or small rib 6 against which the free end of the stem 6 halts, this latter being securely retained within the wall 3 by friction.

At the top (with respect to Figure 1) of the tubular wall 2 there is provided an end wall 7 comprising a circular hole 8.

A profiled element with circular cross-sections (see also Figures 2 to 6) and consisting of two successive portions 9, 10 of different diameters is inserted into and securely housed within the cavity of the tubular wall 2, 3. The portion 9 of the profiled element is forced into and securely retained within the cavity delimited by the tubular wall 3. As this cavity is of lobe shape, the round-section cylindrical portion 9 of the profiled element does not obstruct the entire cavity, but leaves narrow long passages 11 free through which the liquid substance to be dispensed can flow (Figure 3).

The round-section cylindrical portion 10 of the profiled element has a smaller cross-section than the cavity delimited by the tubular wall 2, so that between them an annular cylindrical space 12 is formed (Figure 5) allowing free flow of the substance to be dispensed.

From Figure 1 it can be seen that the portion 10 of the profiled element is longer than the tubular wall portion 2 into which it is inserted (until it abuts against the end wall 7), so that a part of the portion 10 extends into the wider cavity delimited by the wall 3, to hence define an intermediate expansion chamber 13 (see also Figure 4), which is essential for ensuring correct expansion of the pressurized liquid emerging from the stem 4.

From Figure 2 it can be seen that in the upper end (with respect to Figures 1 and 2) of the elongate element 9, 10 there is provided a recess 14 from the centre of which there projects a peg 15 which extends through the centre of the hole 8 provided in the end wall 7, to leave a free annular space for the emergence of the atomized substance. The recess 14 communicates with the annular cylindrical space 12 via channels 16 (see also Figure 6) substantially tangential to the recess periphery, in order to impress on the liquid droplets (originating from the container 5 via the stem 4, the thin long passages 11, the expansion chamber 13, the annular passage 12 and the channels 16) a vortex movement within the annular chamber about the peg 15, the liquid then emerging in finely and uniformly atomized form by rotating about the peg 15 through the space existing between the surface of the peg 15 and the adjacent surface of the hole 8 provided in the end wall 7.

The presence of the intermediate expansion chamber 13 has been found to be very important, this ensuring perfect atomization of the droplets of the substance expelled by the dispenser, as has the maintaining of vortex motion within the dispensed substance by the presence of the peg 15 at the centre of the hole 8.

It will be apparent that the the profiled element 9, 10 and the body 1, 2, 7 can be easily formed without appreciable rejects, due to their very simple structure.

## Claims

1. A nasal dispenser for atomizing pharmaceutical substances, comprising a longitudinal channel having an open end into which the stem (4) of a pump or the like can be inserted, and provided at its other end with a wall (7) provided with a hole (8), said channel housing an elongate profiled element (9, 10) the cross-sections of which are smaller than their surrounding cross-sections of the channel into which said element is inserted, wherein said channel has a cross-section which decreases stepwise towards said wall (7), said element (9, 10) defining within the channel at least one intermediate expansion chamber (13), from the free end of the elongate element (9, 10) in proximity to said wall there projecting a peg (15) which extends through the centre of the hole (8) in said wall (7) to leave a free space for the emergence of the atomized substance, in said end of the elongate element (9, 10) there being provided channels (16) substantially tangential to the peg (15) and opening into the periphery of the elongate element,
characterised in that said profiled element (9, 10) has circular cross-sections decreasing stepwise from one end to the other along its length, and in that the cross-sections of said channel are in the form of lobes within its larger cross-section portion into which said element (9, 10) is forced, the length of that portion (10) of said element (9, 10) inserted into the smaller cross-section portion of said channel being greater than the length of such channel portion.

## Patentansprüche

1. Nasaler Spender zum Zerstäuben von pharmazeutischen Substanzen, umfassend einen Längskanal, der ein offenes Ende hat, in welches der Schaft (4) einer Pumpe oder dergleichen eingefügt werden kann, und der an seinem anderen Ende mit einer mit einem Loch (8) versehenen Wand (7) versehen ist, wobei der Kanal ein langgestrecktes profiliertes Element (9,10) aufnimmt, dessen Querschnitte kleiner als ihre umgebenden Querschnitte des Kanals sind, in welchen das Element eingefügt ist, worin der Kanal einen Querschnitt hat, welcher schrittweise nach der Wand (7) zu abnimmt, wobei das Element (9,10) innerhalb des Kanals wenigstens eine zwischenliegende Expansionskammer (13) begrenzt, wobei von dem freien Ende des langgestreckten Elements (9,10) in der Nähe der Wand ein Zapfen (15) vorsteht, welcher sich durch die Mitte des Lochs (8) in der Wand (7) so erstreckt, daß er einen freien Raum für das Herauskommen der zerstäubten Substanz läßt, wobei in dem Ende des langgestreckten Elements (9,10) Kanäle (16) vorgesehen sind, die im wesentlichen tangential zu dem Zapfen (15) sind, und in den Umfang des langgestreckten Elements münden, dadurch **gekennzeichnet,** daß das profilierte Element (9,10) kreisförmige Querschnitte hat, die schrittweise von einem Ende zu dem anderen längs seiner Länge abnehmen, und daß die Querschnitte des Kanals in der Form von Flügeln innerhalb seines Teils größeren Querschnitts sind, in welchen das Element (9,10) gedrückt ist, wobei die Länge jenes Teils (10) des Elements (9,10), der in den Teil kleineren Querschnitts des Kanals eingefügt ist, größer als die Länge des derartigen Kanalteils ist.

## Revendications

1. Dispensateur nasal pour atomiser des produits pharmaceutiques, comprenant un canal longitudinal avec une extrémité ouverte dans laquelle on peut insérer la tige (4) d'une pompe ou analogue, l'autre extrémité étant fermée par une paroi (7) qui comporte un trou (8), ledit canal logeant un élément profilé allongé (9, 10) dont les sections transversales sont inférieures aux sections transversales environnantes du canal dans lequel est inséré ledit élément, ledit canal ayant une section transversale qui diminue par paliers en direction de ladite paroi (7), ledit élément (9, 10) délimitant, à l'intérieur du canal, au moins une chambre d'expansion intermédiaire (13) une cheville (15) faisant saillie de l'extrémité libre de l'élément allongé (9, 10) au voisinage de ladite paroi (7), cette cheville passant par le centre du trou (8) de la paroi (7) de manière à garder un espace libre permettant la sortie de la substance atomisée, des canaux (16) étant prévus dans l'extrémité de l'élément allongé (9, 10), ces canaux étant sensiblement tangentiels par rapport à la cheville (15) et débouchant dans la périphérie de l'élément allongé, caractérisé en ce que ledit élément profilé (9, 10) présente des sections transversales circulaires qui diminuent par paliers d'une extrémité à l'autre sur toute sa longueur, et en ce que les sections transversales dudit canal ont la forme de lobes à l'intérieur de sa portion à plus grande section transversale dans laquelle on insère par force l'élément (9, 10), la longueur de la portion (10) dudit élément (9, 10) qui est insérée dans la partie à plus petite section transversale du canal étant supérieure à la longueur de ladite partie du canal.
